# EUROPEAN PATENT SPECIFICATION

(11) **EP 2 665 807 B1**
(45) Date of publication and mention of the grant of the patent: **10.10.2018**
(21) Application number: 12837029.3
(22) Date of filing: 29.03.2012
(51) Int. Cl.: C12M 1/00, B03C 1/00, C12M 1/06, C12M 1/12, B01F 3/00, C12M 1/34, C12M 1/36

(54) **MULTIFUNCTIONAL BIOREACTOR SYSTEM AND METHODS FOR CELL SORTING AND CULTURING**
MULTIFUNKTIONELLES BIOREAKTORSYSTEM UND VERFAHREN ZUR ZELLSORTIERUNG UND -KULTIVIERUNG
SYSTÈME DE BIORÉACTEUR MULTIFONCTIONNEL ET PROCÉDÉS POUR LE TRIAGE ET LA CULTURE CELLULAIRES

(30) Priority: 29.03.2011 US 201161468573 P
(43) Date of publication of application: 27.11.2013
(73) Proprietor: Zhang, Yongxin, Carrollton, TX 75007 (US)
(72) Inventor: WANG, Ying, Carrollton, TX 75007 (US); ZHANG, Monica, Carrollton, TX 75007 (US)
(74) Representative: Kramer Barske Schmidtchen Patentanwälte PartG mbB
(86) International application number: PCT/US2012/000182
(87) International publication number: WO 2013/048546

(56) References cited:
- WO-A1-2006/020280
- WO-A2-02/42421
- WO-A2-2005/003332
- WO-A2-2005/010162
- GB-A- 2 400 378
- US-A- 4 154 652
- US-A- 5 026 650
- US-A1- 2005 054 101
- US-A1- 2006 019 385

## Description

### Field of the invention

The invention relates to a multifunctional bioreactor for cell culture and cell sorting.

### Background of the invention

This application relates generally to bioreactors and more particularly bioreactors for growing and separating cells.

Many kinds of cells, especially hematopoietic stem cells and immunocytes, need to be isolated from original samples before they can be efficiently expanded and directed differentiated in culture. This isolation procedure is also called cell sorting or cell separation. Previously, the cell sorting and cell culture have been respectively conducted in separated systems, in which the target cells are isolated first and then are transferred into culture containers. This traditional method is quite cumbersome and has a higher risk for cell contamination and cell loss from the cell sorting to cell culture, in which two completely different devices and systems were involved. Our current invention with a novel design allows these two different procedures completed in one container (chamber), and so minimizes the risk of contamination and target cell loss, and significantly increases the efficiency of the operation.

Some cells are very sensitive to shear-stress in the culture. For example, shear-stress can cause the non-specific differentiation and the increased apoptosis in the stem cell culture, which significantly reduces the efficiency of the stem cell expansion and directed differentiation. The higher shear-stress also causes the more release of non-specific proteins in protein expression, in which the protein of interest takes less proportion in the culture and so result in the increase of protein purification. The static culture has the least shear stress but the cells in static culture normally sit at the bottom of the culture containers, some cells cannot get enough nutrition when cells are at higher density and so not suitable for large scale cell expansion. Some bioreactors, such as NASA's rotation wall vessel (RWV) bioreactor, were designed for reducing the shear-stress. However, these bioreactors have to keep cells in suspension by continuously moving, stirring or/and agitating cells. Once the bioreactor stop running, cells will accumulate somewhere of the bottom but are not evenly distributed, which is harmful for most cell growth. Therefore, though the shear-stress has been reduced in these bioreactors, the reduced shear-stress has to continuously exert on the cultured cells when these bioreactors running. In our current invention, when the bioreactor is at static status, cells are allowed to evenly distribute at the bottom of the culture chamber or on the surface of the magnetic beads. Thus, our invention provides cells the best growth condition in both suspension status and static status.

Some bioreactors use magnet element (specifically blades or vans) controlled by magnet impeller to agitate culture media to keep cells in suspension status. This kind of bioreactor purposely enhances the shear-stress for the culture requirements of a certain cells. In addition to the differences in the application, the bioreactor in our invention does not use blades or canes to be the magnet element, and the magnet beads in our invention actually has no magnetism if they are not in magnet field and they can only gain magnetism when they are placed in magnet field. The magnet beads in our invention are not controlled by impeller but by the changes of magnetic field strength affecting the beads' moving. The proper microenvironments, or so called niches, are very important for the growth of some cells, such as stem cells. Some devices use solid materials to form niches or use gel-like materials to form niches. With these devices, after cells culture, cells need to be rinsed out from niches with a special procedure or the niche-forming materials has to be melted or digested with enzyme to release the target cells. In our current invention, the interspaces among beads naturally from the niches for cell growth, and the cells can be easily released when the beads are lifted by changing the magnet field strength. Some other systems of cell culture attempt to build niches via welled culture plates (such as the common 96-welled plate), micro-chambers (Hung 2005) or micro-sieves (Zhang 2009). The current invented bioreactor is superior to the welled plate because the mobility of the beads creates dynamic microenvironments for cells needing increased flux of medium to grow. It is also superior to micro-chambers and micro-sieves because it poses less of a challenge to manufacture, is easier to sterilize after use, and the size of the niche can be easily modified by adjusting the size of the beads.

Many cell culture containers (chambers) have been designed for bioreactor use, such as common cell culture flasks, gas-permeable bags, rotation wall vessels, and so on. These containers can be used with common perfusion system with which the cells can be diluted and media can be changed. However, during the media change with these containers, the cytokines, proteins and other expensive substances for cell growth and cells' products are removed from the culture simultaneously. And, the efficiency of common dialysis process is not high enough. The cell culture chambers designed in our current invention take the advantage of colloid osmotic force differences between osmosis chambers at two side of culture chamber to allow the media exchange go through the dialysis membranes rapidly without any loss of cytokines, peptides, proteins and other materials in a certain size. This chamber design provides a novel perfusion strategy and the system with it is called as gradient osmosis perfusion system.

Most bioreactors were designed for culturing either adherent or suspension cells. No bioreactors have been reported to support the growth of partial adherent cells yet. The bioreactor in our current invention can be used for the culture of almost any cell, including suspension cells, adherent cells and partial adherent cells.

It is very common to use computer to control bioreactor running, and many bioreactors are programmable. It is emphasized in our current invention that (1) the strength and direction of magnetic fields, (2)the frequencies and speeds of cell culture chamber flip, and (3) frequencies and speeds of the magnet beads as results of above (1) and (2) are controlled by pre-selected programs or/and programs that response to the data it receives from detectors and send feedback to bioreactor. Similar to the application of computer in bioreactor control, several cell density detectors with some special light sources (such as laser projector) have been designed for monitoring the cell concentration during the cell culture. The data obtained from the detector or sensor is used to determine if the cells need to be diluted if the culture needs media change. In our current invented bioreactor, the cell density detector uses the common light source and the data is specifically used to adjust the strength of the magnetic field, the flipping speed and frequency of cell culture chamber, as well as indirectly adjust the moving speed and frequency of the magnet beads.

WO 2005/010162 A2 relates to a bioreactor suitable for growing cells comprising (a) a culture vessel comprising at least one engineered microcarrier comprising at least one cell and culture medium sufficient for growth of said cell, and (b) at least one source for generating at least one physical force to which said microcarrier is responsive.

WO 2005/003332 A2 relates to a system for cultivating three-dimensional biological cells, the system comprising two diamagnet plates, an upper lifter magnet and a culture chamber containing a culture medium and a plurality of bioattractive magnetized core particles, the culture chamber further comprising an influx port and an outflux port, the culture chamber positioned between the diamagnet plates relative to the upper lifter magnet, to facilitate levitation of the magnetized core particles, and a plurality of biological cells to be cultivated, the cells placed within the culture chamber and adhering to the magnetized core particles.

GB 2 400 378 A relates to a method for culturing cells, comprising the steps of preparing a cell culture solution containing at least cells to be cultured and granular cell culture carriers to which the cells are allowed to adhere and grow thereon, and applying a magnetic field to the cell culture solution so as to agitate the cell culture solution by the effect of the magnetic field, whereby the cells adhere to and grow on the surfaces of the cell culture carriers.

### Summary

Bioreactors are typically employed to grow cells within a culture. However, many kinds of cells need to be isolated from original samples before they can be efficiently expanded and directed differentiated in culture. This isolation procedure is also called cell sorting or cell separation. Typically, the cell sorting and cell culture have been conducted in separated systems, in which the target cells are isolated first and then are transferred into culture containers. This traditional method is quite cumbersome and has a higher risk for cell contamination and cell loss from the cell sorting to cell culture, in which two completely different devices and systems are involved.

Additionally, many bioreactors employ rotating impellers or the like for mixing the contents of the bioreactor. Unfortunately, this imparts a shear stress on the cells which may damage the cells, reduces the efficiency of the system, causes a release of waste products, such as non-specific proteins expression, or the like. Additionally, in some bioreactors, cells may aggregate on the bottom of the cell culture chamber or other locations within the cell culture chamber. The aggregation of these cells in the chamber is not conducive to efficient cell growth. Therefore, there exists a significant need for an efficient bioreactor capable of growing and separating cells therewithin while also minimizing shear-stress imparted to the cells.

The present invention satisfies this need by a bioreactor system as claimed in claim 1. Further embodiments of the present invention are set out in the dependent claims.

### Brief Description of the Drawings

The drawings, when considered in connection with the following description, are presented for the purpose of facilitating an understanding of the subject matter sought to be protected.
FIG. 1 is a schematic view showing an illustrative bioreactor;
FIGS. 2a-2f show a series of schematic views of a bioreactor showing the movement of an agitator within a chamber in series;
FIG. 3 is a schematic isometric view of a chamber;
FIG. 4 is a schematic side-view of a chamber; and
FIG. 5 is a schematic view of an agitator.

### Detailed Description

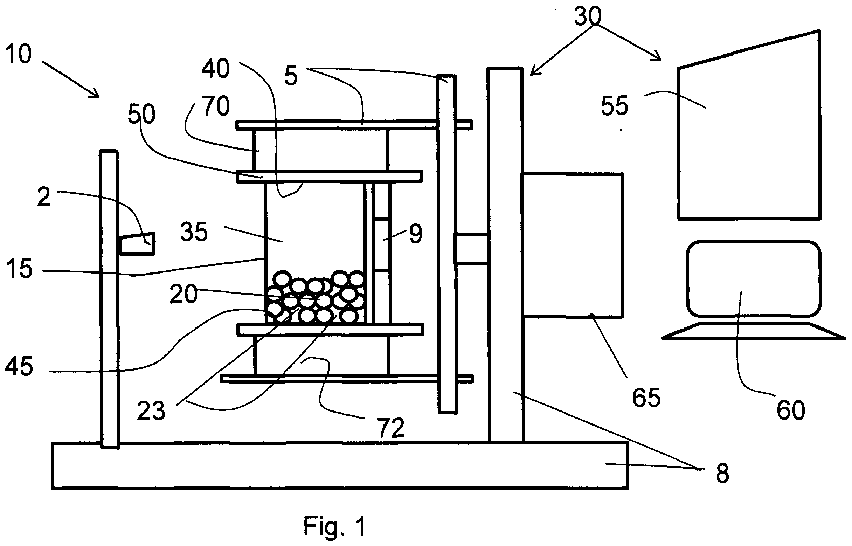

Referring now to FIG. 1, a bioreactor system 10 for growing and separating cells is shown. The system 10 includes a cell culture chamber 15, an agitator 20 and a control system 30.

The cell culture chamber 15 includes an interior 35 for receiving and growing target cells in a cell culture media disposed therein, a first end 40 and a second end 45. As used herein, "target cells" refers to cells disposed within the chamber 15 and which are grown within the chamber 15. While the present disclosure is given the context of growing target cells, it will be appreciated that the system may be employed to mix chemicals or any other suitable solution or material. Also, while the first end 40 and second end 45 are shown as being at the top and bottom of the chamber 15 respectively, it will be appreciated that the ends 40, 45 may be in any suitable orientation relative to one another (e.g., in a horizontal plane) and remain within the scope of the present disclosure. As will be discussed below, the chamber 15 includes three interior compartments. In addition, as will be appreciated by those skilled in the art, the chamber 15 may be formed from any suitable material, including a rigid material, a flexible material, a combination of rigid and flexible materials, a gas permeable material or any other suitable material. The chamber 15 may further include one or more ports for providing fluid communication between the chamber interior 35 and one or more reservoirs. Illustrative reservoirs include, without limitation, a cell culture media reservoir, a waste reservoir, a buffer reservoir, a CO₂ reservoir, or any other suitable reservoir.

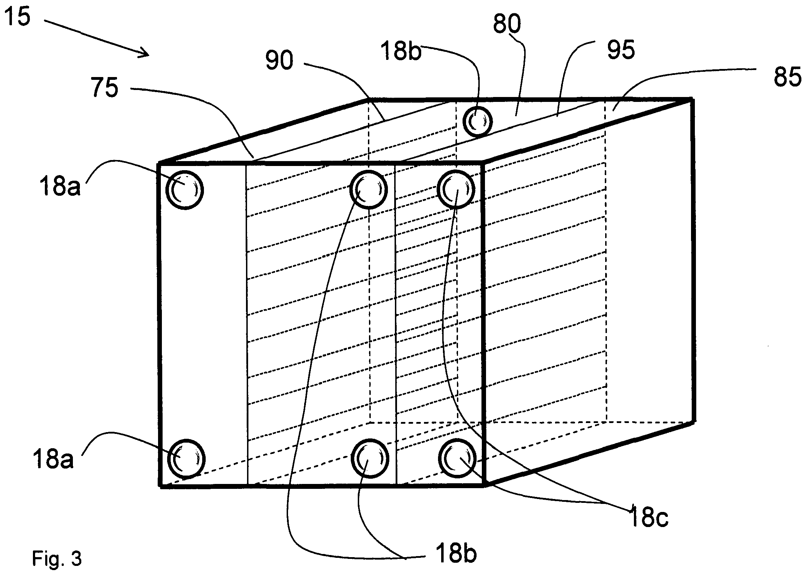

Referring now to FIG. 3, a chamber 15 according to the present invention is shown. In this embodiment, the chamber 15 includes a first compartment 75, a second compartment 80 and a third compartment 85. The first compartment 75 may include one or more ports 18a for providing fluid communication between the first compartment and one or more reservoirs such as: a cell culture media reservoir, such that media can be added to and/or removed from the first compartment; a buffer reservoir, such that buffer can be added to and/or removed from the first compartment; a waste reservoir, such that waste may be removed from the first compartment 75; a CO₂ reservoir such that CO₂ may be added to and/or removed from the first compartment; or any other suitable reservior. The first compartment 75 and second compartment 80 are separated by a first membrane 90. The first membrane 90 is adapted to selectively permit media to flow from the first compartment 75 to the second compartment 80. The membrane 90 may include perforations, apertures or any other suitable configuration and/or is otherwise semipermeable so as to permit the media to flow from the first compartment 75 to the second compartment 80. In one embodiment, the membrane 90 is a dialysis membrane. The membrane 90 may be formed from any suitable material, including but not limited to cellulose and cellophane.

The second compartment 80 is configured to retain the agitator therein such that the agitator may be moved within the compartment as previously discussed relative to FIG. 1. Target cells are typically grown and retained within the second compartment 80 as well. The second compartment 80 may include one or more ports 18b for providing fluid communication between one or more reservoirs such as the types of reservoirs discussed relative to the first compartment 75. The second compartment 80 and third compartment 85 may be separated by a second membrane 95. The second membrane 95 is adapted to selectively permit media and/or waste to flow from the second compartment 75 to the third compartment 85. The membrane 95 may include perforations, apertures or any other suitable configuration and/or is otherwise semipermeable so as to permit the media and/or waste to flow from the second compartment 80 to the third compartment 85. In one embodiment, the membrane 95 is a dialysis membrane. The membrane 95 may be formed from any suitable material, including but not limited to cellulose and cellophane. The third compartment 85 may include one or more ports 18c for providing fluid communication between the third compartment 85 and one or more reservoirs such as the types of reservoirs discussed relative to the first compartment 75. Also, in at least one embodiment, at least a portion of the exterior of the chamber may be formed from a gas permeable material. When at least a portion of the chamber exterior is formed from a gas permeable material the system may, in some but not necessarily all, instances not include direct gas injection into the chamber and media pH maintaining components. The first compartment 75 and third compartment 85 may be located in any suitable position relative to the second compartment 80 and the present disclosure is not in any way limited to the second compartment 80 positioned between the first and third compartments 75, 80. Additionally, it will be appreciated that any suitable number of compartments may be employed and the present disclosure is in not limited to only three compartments.

In one embodiment, a media comprising a suitable solution having a relatively small number of large and/or large polar molecules is provided to the first compartment 75, and the same or similar media with suitable colloid osmosis for cell growth is provided to the second compartment 80. Suitable media may be any media that used for growing or maintaining the target cells. A buffer having a greater concentration of large and/or large polar molecules to generate and maintain higher colloid osmosis force, as compared to the media of the first and/or second compartments 75, 80, is provided to the third compartment 85. The materials used to generate and maintain higher colloid osmosis force for the third compartment 85 may include, but is not limited to, PEG 80000, albumin, and other proteins or any other suitable material or solution. The membranes 90, 95 may have the same or different permeability and are configured such that media and/or waste from the growth of new cells may flow from the first and/or second chambers 75, 80 to the third chamber 85 via osmosis. The control system may control the infusion and draining of media, buffer and/or waste from one or more compartments 75, 80, 85 so as to maintain a constant volume and osmosis force in each compartment 75, 80, 85 and maintain a supply of fresh media to the second compartment 80. In addition, the control system, via any suitable detection device, mechanism or method, may monitor the any suitable parameter involved in the growth of the target cells, for example and without limitation, the change in the number of target cells, pH, CO₂, glucose, calcium, potassium, sodium, temperature, humidity or any other suitable factor and adjust the interval, frequency and/or speed of the movement of the agitator within the second chamber and/or adjust the amount of media, the type of media, the amount of buffer, the type of buffer, the amount of CO₂, or make any other suitable adjustment based on the control system measurement so as to enhance or promote the growth of the target cells within the chamber.

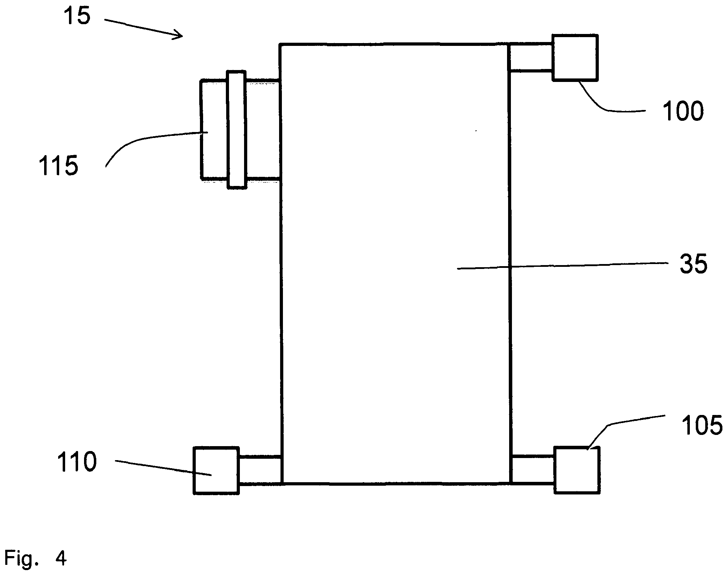

Referring now to FIG. 4, another illustrative chamber 15 is shown. In this embodiment, the chamber may include one or more ports 100 for providing fluid communication between the chamber interior 35 and a waste reservoir. The chamber 15 may also include one or more ports 110 for providing fluid communication between the chamber interior 35 and a cell culture media reservoir. Also, the chamber 15 may include one or more ports 110 for providing fluid communication between the chamber interior 35 and a buffer reservoir. Further, the chamber may also include one or more ports 115 for receiving the agitator into the chamber interior 35. In one embodiment, the chamber 15 is formed from Teflon FEP. This particular embodiment may be useful in instances where the target cells will adhere or otherwise be coupled to the agitator so that when waste is flushed from the chamber interior 35, the target cells remain within the chamber interior 35. However, it will be appreciated that the chamber 15 may be formed from any suitable material and remain within the scope of the present disclosure.

The agitator 20 is disposed within the chamber interior 35 and is capable of moving between the chamber first end 40 and chamber second end 45. Alternatively, the agitator 20 may be configured to be moved between any two or more points, or between any two or more portions, within the chamber interior 35. In the illustrative embodiment, the agitator comprises a plurality of beads 21. It will be appreciated that any illustrative embodiment showing beads 21 may use any alternative agitator configuration and remain with the scope of the present disclosure and that any particular illustrative embodiment is not limited to using beads exclusively as the agitator. In one embodiment, the beads 21 are be formed from a magnetizable material, such as silicon steel, Fe₃O₄, or any other suitable magnetizable material. As used herein, magnetizable means that the agitator, such as the beads, will hold a magnetic charge when subjected to a magnetic field but will not otherwise hold a magnetic charge once removed from the magnetic field, or the magnetic field removed from the vicinity of the agitator, for example, when a magnetic field generator is de-energized. The magnetizable material typically comprises the core of each bead 21. The magnetizable core may then be coated with any suitable material. In one embodiment, the magnetizable core is coated with polystyrene; however, it will be appreciated that the magnetizable core may be coated with any suitable material and remain within the scope of the present disclosure. For example, and without limitation, the magnetizable core may be coated with any suitable thermoplastic or thermoset polymer. While the beads 21 are shown as being formed from a magnetizable material, it will be appreciated that the beads may be formed from any suitable material, magnetizable or non-magnetizable, and remain within the scope of the present disclosure. Additionally, it will be appreciated that the beads 21 may each be coated with any suitable material such that the target cells will adhere to the beads as the cells grow within the chamber 15, yet it will be appreciated that beads not coated with a particular material to which target cells will adhere also remain within the scope of the present disclosure. In some embodiments, it may be desirable to have beads 21 that are buoyant within the cell culture media; therefore, the core of the beads may include air pockets or bubbles, a lightweight foam or plastic or any other suitable material for permitting the beads 21 to be buoyant within the media.

The beads 21 may be formed such that one or more niches, or micoenvironments, may be formed or created in the voids between the beads 21 when the beads are stacked together. In some embodiments, these niches may promote growth of additional target cells therein. In one embodiment, where the beads are substantially spherical, the diameter of each bead 21 may be between 1mm and 10mm for the creation of suitable niches. However, it will be appreciated that the beads 21 may have any suitable size and/or shape such that one or more suitable niches may be formed when the beads 21 are stacked together. Also, it will be appreciated that at least some niches may be formed between some beads and one or more walls of the chamber interior.

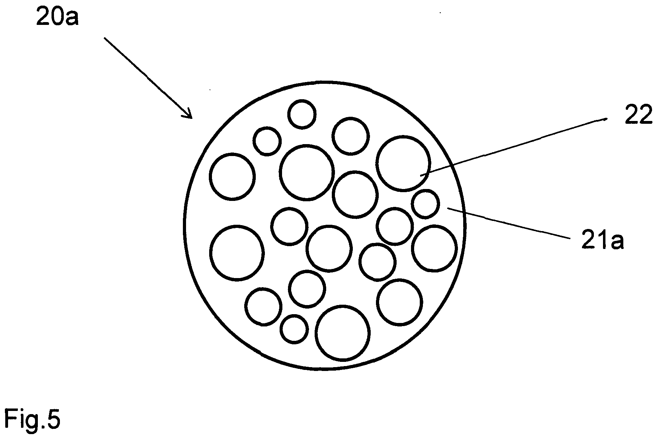

In an alternative embodiment, as shown in FIG. 5, the agitator 20a may be a plate member 21a having a plurality of apertures 22 therein. The cross-section of the plate member 21a may be complimentary to the cross-sectional shape of the chamber 15 such that the agitator 20a may move within the chamber interior 35. The apertures 22 may permit the media to flow through the agitator 20a as the agitator 20a moves within the chamber interior 35. The agitator 20a may be formed from magnetizable materials or non-magnetizable material, formed to be buoyant or non-buoyant, and/or coated as previously discussed with respect to the beads 20.

Referring again to FIG. 1, the control system 30 may include one or both of a controller 55 and computer 60 for controlling operation of the system 10. Alternatively, the system 10 may be run manually. The control system 30 is configured to be releasably coupled to the chamber 15. The control system 30 may include a cassette 50 for receiving the chamber 15 but it will be appreciated that the chamber 15 may be coupled to the control system 50 via any suitable means or configuration (e.g., clips, hooks, magnets, hook-and-loop assemblies, friction fit, etc.) and remain within the scope of the present disclosure.

The control system 30 may also include a light source 2 and a cell detector 9 for detecting the number of cells within the chamber 15, detecting the change in the number of cells within the chamber 15 or the like and reporting the results back to the control system 30. However, it will be appreciated that any detector, mechanism or technique known in the art for monitoring the number of cells or the change in the number of cells may be employed and remain within the scope of the present disclosure. Additionally, the control system 30, via any suitable detection device, mechanism or method, may monitor the any suitable parameter involved in the growth of the target cells, for example and without limitation, the change in the number of target cells, pH, CO₂, glucose, calcium, potassium, sodium, temperature, humidity or any other suitable factor and adjust the frequency and/or speed of the movement of the agitator within the chamber and/or adjust the amount of media, the type of media, the amount of buffer, the type of buffer, the amount of CO₂, or make any other suitable adjustment based on any control system measurement so as to enhance or promote the growth of the target cells within the chamber 15.

The control system 30 is operable to cause the agitator to move within the interior 35 of the chamber 15. This may be accomplished a variety of ways. In the illustrative embodiment, the control system includes a motor 65 operable to rotate the chamber 15 between a first position and second position. As will be discussed below, the first position and second position are approximately 180° apart but it will be appreciated that first and second positions may have any suitable angular relationship relative to one another and remain within the scope of the present disclosure. The chamber 15 may be rotated in a horizontal plane, rotated in a vertical plane or rotated, shifted, slid or otherwise moved in any suitable manner to cause the agitator 20 to move within the chamber 15.

In addition, the control system 30 may include first and second magnetic field generators 70, 72 for exciting the beads 21, or other agitator 20, so as to move the beads 21 within the chamber 15 to mix the target cells and culture media. In the illustrative embodiment, each magnetic field generator is an electromagnet that generates a magnetic field when energized and ceases to create a magnetic field when de-energized. When energized, each magnetic field generator draws the agitator 20, e.g. the beads 21, toward the energized magnetic field generator. In an alternative embodiment, a permanent magnet may be used wherein the control system 30 is operable to remove the magnet from the vicinity of the chamber 15 or otherwise block the magnetic field from the magnet from penetrating into the chamber 15. While the illustrative embodiment employs both chamber rotation and electromagnets for moving the agitator within the chamber, it will be appreciated that chamber rotation may be used alone or that electromagnets may be used alone. Moreover, it will be appreciated that any technique for moving the agitator within the chamber may be employed and remain within the scope of the present disclosure.

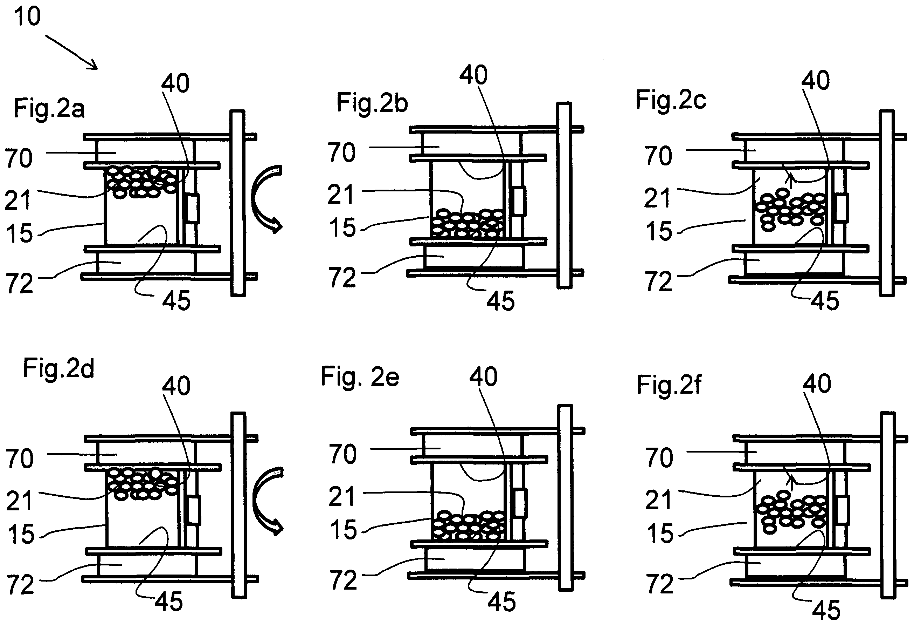

Referring now to FIG. 2a-2f, operation of the system 10 is illustrated by way of a non-limiting example. Target cells and cell culture media are delivered to the interior 35 of the chamber 15. In this embodiment, the beads 21 are buoyant and float near the top of the cell culture media within the chamber 15. In FIG. 2a, the first magnetic field generator 70 is energized and the beads 21 are held near the first end 40 of the chamber 15. The chamber 15 is then rotated approximately 180° to a position as shown in FIG. 2b wherein the first magnetic field generator 70 maintains the beads 21 near the chamber first end 40. The first magnetic field generator 70 may then be de-energized whereby the beads 21 begin to float towards the second end 45 of the chamber 15 as shown in FIG. 2c. In embodiments where the beads 21 include a coating which target cells will adhere to, movement of the beads 21 from one end to the other will collect newly grown target cells. The target cells may adhere to the beads 21 while waste is flushed from the chamber 15 and/or when new media is introduced to the chamber 15 such that a substantial number of the target cells, original and newly grown, remain within the chamber. Alternatively, magnetizable antibodies specific to the target cells may be added to the interior of chamber 15 whereby the antibodies will bind themselves to the target cells, and when a magnetic field is introduced to the chamber, the antibody bound target cells will be releasably coupled to the magnetizable beads 21 and/or the chamber wall(s) adjacent to the magnetic field generator(s). In this embodiment, one or both of the magnetic field generators 70, 72 may remain energized while unbound cells and/or waste are flushed from the chamber and/or while new media is introduced to the chamber such that a substantial number of the target cells, original and newly grown, remain within the chamber 15. Alternatively, magnetic reagents, such as Annexin V or other suitable reagent, may be employed to couple to damaged or dead cells to the beads and the healthy target cells flushed from the system 10. Further, it will be appreciated that magnetizable antibodies and/or reagents may be employed in a chamber 15 without the use of an agitator whereby the target cells or damaged/dead cells may be held against the chamber when the chamber is flushed.

Referring again to FIGS., once the beads 21 are near the second end 45 of the chamber, the second magnetic field generator 72 may be energized whereby the beads 21 are held near the chamber second end 45 (FIG 2d) and the chamber rotated to the position shown in FIG. 2e. The second magnetic field generator 72 may then be de-energized whereby the beads 21 will float towards the chamber first end 40 as shown in FIG. 2f. As will be appreciated by those skilled in the art, a variety of additives, media, buffers, CO₂ and the like may be selectively added to the chamber at any desired point during this process and/or waste selectively removed in order to promote or enhance new cell growth based on measurements taken by the control system as previously discussed.

In an alternative embodiment, non-buoyant beads may be employed such that the beads are moved within the chamber by rotation of the chamber and without also being subjected to magnetic fields. Here, gravity and centrifugal force, by way of rotation of the chamber, are employed to move the beads between two or more points within the chamber 15. In yet another alternative, the first and second magnetic field generators 70, 72 may be alternately energized so as to move the beads between two or more points within the chamber and without any rotation of the chamber 15. While the forgoing example employs beads 21 as the agitator, it will be appreciated that suitable device may be employed as the agitator and remain within the scope of the present disclosure, including but not limited to that of FIG. 5. Moreover, it will be appreciated that any means or technique for moving the agitator within the chamber may be employed and remain within the scope of the present disclosure.

Moreover, it will be appreciated that if the chamber 15 formed from gas permeable material or otherwise includes a gas permeable portion, the system may be disposed within a CO₂ incubator or CO₂ room. Without a CO₂ incubator or CO₂ room or without any gas permeable portion of the chamber, reagents, such as HEPES may be employed or, alternatively, CO₂ may be injected directly into the chamber from a CO₂ reservoir.

## Claims

1. A bioreactor system (10) for separating cells based on cell markers and growing cells and engineered tissue comprising at least:
one reaction chamber (15) comprising an interior (35) having a first portion and a second portion, and providing a space for the reaction of cell marker-based cell separation, cell growth and engineered tissue growth;
a magnetizable agitator (20, 20a) disposed within the chamber interior and capable of moving between the interior first portion and interior second portion;
an adjustable magnetic field operable to support the cell marker-based cell separation and to cause the agitator (20, 20a) to stay at a certain interior position of the chamber (15) or to move between the interior first portion and interior second portion accompanied with the inversion of the reaction chamber (15),
wherein the reaction chamber interior (35) comprises:
a first compartment (75) as a low osmosis compartment, the first compartment (75) adapted for fluid communication with a cell culture media reservoir such that the first compartment (75) is capable of receiving and depleting cell culture media from the reservoir;
a second compartment (80) as a reaction compartment adjacent to the first compartment (75) and also adapted for ports (18b) for receiving and depleting cells, agitators, reagents, buffer and media, wherein the agitator (20, 20a) is disposed within the second compartment (80);
a first membrane (90) positioned between the first compartment (75) and second compartment (80), the first membrane (90) adapted to selectively permit cell culture media to flow from the first compartment (75) to the second compartment (80);
a third compartment (85) as a high osmosis compartment adjacent to the second compartment (80), the third compartment (85) adapted for fluid communication with a buffer reservoir such that the third compartment (85) is capable of receiving buffer from the buffer reservoir and wherein the third compartment (85) is further adapted for fluid communication with a waste reservoir such that waste is capable of flowing from the third reservoir (85) to the waste reservoir;
wherein the first compartment (75) contains a lower concentrated colloid osmotic solution compared to the media of the third compartment (85);
a second membrane (95) positioned between the second compartment (80) and third compartment (85), the second membrane (95) adapted to selectively permit flow of media and waste from the second compartment (80) to the third compartment (85); and
at least one part of the reaction chamber side wall made with gas permeable materials, one component of media containing reagent for keeping pH, or a gas-permeable system in connection to the chamber (15), or any combination of the above three.

2. The bioreactor system (10) of claim 1, wherein the magnetizable agitators (20, 20a) comprise a plurality of beads (21) or a plate member (21a) having a plurality of apertures (22) and are made
(1) in whole with rigid magnetizable materials and are either uncoated or coated with inert and rigid or hard materials for any purpose including attachment of cells and reducing cell damage; or
(2) at least partially with magnetizable material which becomes magnetic when placed inside a magnetic field but is not magnetic or very weakly magnetic when the magnetic field disappears or is removed from the reaction area; and
(3) in a size greater than 1 millimeter in diameter for beads (21) and smaller than the volume of said reaction chamber (15).

3. The bioreactor system (10) of claim 1, wherein said adjustable magnetic field can be generated (1) by an electric magnet which adjusts the magnetic field by changing its electric current and/or voltage or by changing the position and direction of the electric magnet; (2) by a permanent magnet that alters the magnetic field by changing the position and direction of the permanent magnet; or (3) by the combination of the above (1) and (2).

4. The bioreactor system (10) of claim 1, further comprising a control system (30) including a computerized program and feedback system capable of automatically adjusting the magnetic field, perfusion, reaction chamber inversion and agitator movement of the bioreactor using a preselected program or being based on the signals or information obtained by its detectors and sensors from the reaction environment.

5. The bioreactor system (10) of claim 4, wherein at least one part of the reaction chamber (15) is coupled to the control system (30) via a cassette (50) of the control system (30) and any holder of the bioreactor.

6. The bioreactor system (10) of claim 1, wherein said adjustable magnetic field can be adjusted by changing the strength, direction or position of the magnetic field via a control system during the cell marker-based cell separation, the cell growth and the engineered tissue growth.

## Patentansprüche

1. Bioreaktorsystem (10) zum Trennen von Zellen auf der Basis von Zellmarkern und zum Wachsenlassen von Zellen und technisch hergestelltem Gewebe, umfassend mindestens:
eine Reaktionskammer (15), die ein Inneres (35) mit einem ersten Abschnitt und einem zweiten Abschnitt umfasst, und die einen Raum für die Reaktion einer Zellmarker-basierten Zelltrennung, ein Zellwachstum und ein Wachstum von technisch hergestelltem Gewebe bereitstellt;
einen magnetisierbaren Rührer (20, 20a), der innerhalb des Inneren der Kammer angeordnet ist und sich zwischen dem ersten Abschnitt des Inneren und dem zweiten Abschnitt des Inneren bewegen kann;
ein einstellbares Magnetfeld, das zum Unterstützen der Zellmarker-basierten Zelltrennung und zum Bewirken betrieben werden kann, dass der Rührer (20, 20a) an einer Position des Inneren der Kammer (15) verbleibt oder sich zwischen dem ersten Abschnitt des Inneren und dem zweiten Abschnitt des Inneren einhergehend mit der Inversion der Reaktionskammer (15) bewegt,
wobei das Innere (35) der Reaktionskammer umfasst:
eine erste Kammer (75) als Kammer mit geringer Osmose, wobei die erste Kammer (75) für eine Fluidverbindung mit einem Zellkulturmedienreservoir angepasst ist, so dass die erste Kammer (75) Zellkulturmedien von dem Reservoir erhalten und aufbrauchen kann;
eine zweite Kammer (80) als Reaktionskammer angrenzend an die erste Kammer (75) und die auch für Öffnungen (18b) zum Erhalten und Aufbrauchen von Zellen, Rührern, Reagenzien, Puffern und Medien angepasst ist, wobei der Rührer (20, 20a) innerhalb der zweiten Kammer (80) angeordnet ist;
eine erste Membran (90), die zwischen der ersten Kammer (75) und der zweiten Kammer (80) angeordnet ist, wobei die erste Membran (90) angepasst ist, um es Zellkulturmedien selektiv zu ermöglichen, von der ersten Kammer (75) zu der zweiten Kammer (80) zu strömen;
eine dritte Kammer (85) als Kammer mit starker Osmose angrenzend an die zweite Kammer (80), wobei die dritte Kammer (85) für eine Fluidverbindung mit einem Pufferreservoir angepasst ist, so dass die dritte Kammer (85) Puffer von dem Pufferreservoir erhalten kann und wobei die dritte Kammer (85) ferner für eine Fluidverbindung mit einem Abfallreservoir angepasst ist, so dass Abfall von dem dritten Reservoir (85) zu dem Abfallreservoir strömen kann;
wobei die erste Kammer (75) verglichen mit den Medien der dritten Kammer (85) eine niedriger konzentrierte osmotische Kolloidlösung enthält;
eine zweite Membran (95), die zwischen der zweiten Kammer (80) und der dritten Kammer (85) angeordnet ist, wobei die zweite Membran (95) zum selektiven Ermöglichen eines Strömens von Medien und Abfall von der zweiten Kammer (80) zu der dritten Kammer (85) angepasst ist; und
mindestens ein Teil der Wand auf der Seite der Reaktionskammer aus gasdurchlässigen Materialien, einer Komponente von Medien, die ein Reagenz zum Beibehalten des pH-Werts enthält, oder einem gasdurchlässigen System in Verbindung mit der Kammer (15) oder jedweder Kombination der vorstehenden drei hergestellt ist.

2. Bioreaktorsystem (10) nach Anspruch 1, bei dem die magnetisierbaren Rührer (20, 20a) eine Mehrzahl von Kügelchen (21) oder ein Plattenelement (21a) mit einer Mehrzahl von Öffnungen (22) umfassen und hergestellt sind aus
(1) im Ganzen aus starren, magnetisierbaren Materialien und entweder unbeschichtet oder mit inerten und starren oder harten Materialien für jedweden Zweck, einschließlich einem Anbringen von Zellen und einem Vermindern einer Zellbeschädigung, beschichtet sind; oder
(2) mindestens teilweise aus einem magnetisierbaren Material, das magnetisch wird, wenn es innerhalb eines Magnetfelds angeordnet ist, jedoch nicht magnetisch oder sehr schwach magnetisch ist, wenn das Magnetfeld verschwindet oder aus dem Reaktionsbereich entfernt wird; und
(3) in einer Größe eines Durchmessers von mehr als 1 Millimeter für Kügelchen (21) und kleiner als das Volumen der Reaktionskammer (15) vorliegen.

3. Bioreaktorsystem (10) nach Anspruch 1, bei dem das einstellbare Magnetfeld erzeugt werden kann durch (1) einen Elektromagneten, der das Magnetfeld durch Verändern von dessen elektrischem Strom und/oder Spannung oder durch Verändern der Position und der Richtung des Elektromagneten verändert; (2) durch einen Permanentmagneten, der das Magnetfeld durch Verändern der Position und der Richtung des Permanentmagneten verändert; oder (3) durch die Kombination der vorstehenden (1) und (2).

4. Bioreaktorsystem (10) nach Anspruch 1, das ferner ein Steuersystem (30) umfasst, das ein computergestütztes Programm und Regelungssystem umfasst, welches das Magnetfeld, die Perfusion, die Reaktionskammerinversion und die Rührerbewegung des Bioreaktors unter Verwendung eines vorausgewählten Programms oder auf der Basis von Signalen oder Informationen, die durch dessen Detektoren und Sensoren von der Reaktionsumgebung erhalten werden, automatisch einstellen kann.

5. Bioreaktorsystem (10) nach Anspruch 4, bei dem mindestens ein Teil der Reaktionskammer (15) mittels einer Kassette (50) des Steuersystems (30) und jedwedem Halter des Bioreaktors mit dem Steuersystem (30) gekoppelt ist.

6. Bioreaktorsystem (10) nach Anspruch 1, bei dem das einstellbare Magnetfeld durch Verändern der Stärke, der Richtung oder der Position des Magnetfelds mittels eines Steuersystems während der Zellmarker-basierten Zelltrennung, des Zellwachstums und des Wachstums des technisch hergestellten Gewebes eingestellt werden kann.

## Revendications

1. Système de bioréacteur (10) pour séparer des cellules sur la base de marqueurs cellulaires et de cellules en croissance et de tissu artificiel comprenant au moins :
une chambre de réaction (15) comprenant un intérieur (35) ayant une première partie et une seconde partie, et fournissant un espace pour la réaction de séparation cellulaire basée sur un marqueur cellulaire, la croissance cellulaire et la croissance tissulaire artificielle ;
un agitateur magnétisable (20, 20a) disposé à l'intérieur de la chambre et capable de se déplacer entre la première partie intérieure et la deuxième partie intérieure ;
un champ magnétique réglable utilisable pour supporter la séparation cellulaire basée sur un marqueur cellulaire et pour amener l'agitateur (20, 20a) à rester dans une certaine position intérieure de la chambre (15) ou à se déplacer entre la première partie intérieure et la deuxième partie intérieure accompagnée de l'inversion de la chambre de réaction (15),
dans lequel l'intérieur de la chambre de réaction (35) comprend :
un premier compartiment (75) en tant que compartiment à faible osmose, le premier compartiment (75) agencé pour une communication de fluide avec un réservoir de milieux de culture cellulaire tel que le premier compartiment (75) est capable de recevoir et d'épuiser des milieux de culture cellulaire du réservoir;
un deuxième compartiment (80) en tant que compartiment de réaction adjacent au premier compartiment (75) et également agencé pour des orifices (18b) pour recevoir et épuiser les cellules, les agitateurs, les réactifs, les tampons et les milieux, où l'agitateur (20, 20a) est disposé dans le deuxième compartiment (80) ;
une première membrane (90) positionnée entre le premier compartiment (75) et le deuxième compartiment (80), la première membrane (90) agencée pour permettre sélectivement aux milieux de culture cellulaire de s'écouler du premier compartiment (75) au deuxième compartiment (80) ;
un troisième compartiment (85) en tant que compartiment à osmose élevée adjacent au deuxième compartiment (80), le troisième compartiment (85) agencé à la communication de fluide avec un réservoir tampon de manière à ce que le troisième compartiment (85) est capable de recevoir du tampon provenant du réservoir tampon et où le troisième compartiment (85) est en outre agencé pour la communication de fluide avec un réservoir de déchet tel que le déchet peut s'écouler du troisième réservoir (85) vers le réservoir de déchet ;
dans lequel le premier compartiment (75) contient une solution osmotique colloïdale de concentration inférieure par rapport aux milieux du troisième compartiment (85) ;
une deuxième membrane (95) positionnée entre le deuxième compartiment (80) et le troisième compartiment (85), la deuxième membrane (95) agencée pour permettre sélectivement un écoulement de milieux et déchet du deuxième compartiment (80) au troisième compartiment (85) ; et
au moins une partie de la paroi latérale de la chambre de réaction réalisée avec des matériaux perméables aux gaz, un composant de milieux contenant un réactif pour maintenir le pH, ou un système perméable aux gaz en connexion avec la chambre (15), ou toute combinaison des trois précédentes.

2. Système de bioréacteur (10) selon la revendication 1, dans lequel les agitateurs magnétisables (20, 20a) comprennent une pluralité de billes (21) ou un élément de plaque (21a) ayant une pluralité d'ouvertures (22) et sont faits
(1) en entier avec des matériaux magnétisables rigides et sont soit non revêtus soit revêtus avec des matériaux inertes et rigides ou dur pour n'importe quel but y compris une fixation des cellules et une réduction de dommage cellulaire ; ou
(2) au moins partiellement avec un matériau magnétisable qui devient magnétique lorsqu'il est placé à l'intérieur d'un champ magnétique mais n'est pas magnétique ou très faiblement magnétique lorsque le champ magnétique disparaît ou est retiré de la zone de réaction ; et
(3) dans une taille supérieure à 1 millimètre de diamètre pour des billes (21) et plus petit que le volume de ladite chambre de réaction (15).

3. Système de bioréacteur (10) selon la revendication 1, dans lequel ledit champ magnétique réglable peut être généré (1) par un aimant électrique qui ajuste le champ magnétique en changeant son courant et/ou tension électrique ou en changeant la position et la direction de l'aimant électrique ; (2) par un aimant permanent qui modifie le champ magnétique en changeant la position et la direction de l'aimant permanent ; ou (3) par la combinaison de ce qui précède (1) et (2).

4. Système de bioréacteur (10) selon la revendication 1, comprenant en outre un système de commande (30) comprenant un programme informatisé et un système de rétroaction capable de régler automatiquement le champ magnétique, la perfusion, l'inversion de la chambre de réaction et le mouvement de l'agitateur du bioréacteur en utilisant un programme présélectionné ou en se basant sur les signaux ou les informations obtenus par ses détecteurs et capteurs de l'environnement de réaction.

5. Système de bioréacteur (10) selon la revendication 4, dans lequel au moins une partie de la chambre de réaction (15) est couplée au système de commande (30) via une cassette (50) du système de commande (30) et tout détenteur du bioréacteur.

6. Système de bioréacteur (10) selon la revendication 1, dans lequel ledit champ magnétique réglable peut être réglé en changeant la force, la direction ou la position du champ magnétique via un système de commande au cours de la séparation cellulaire basée sur un marqueur cellulaire, la croissance cellulaire et la croissance de tissu artificiel.
